# EUROPÄISCHE PATENTANMELDUNG

(11) **EP 1 338 238 A2**
(43) Veröffentlichungstag der Anmeldung: **27.08.2003**
(21) Anmeldenummer: 03001157.1
(22) Anmeldetag: 21.01.2003
(51) Int. Cl.: A61B 3/13

(54) **Ophthalmo-Operationsmikroskop**

(30) Priorität: 23.01.2002 DE 10202509
(71) Anmelder: Leica Microsystems (Schweiz) AG, 9435 Heerbrugg (CH)
(72) Erfinder: Sander, Ulrich, Dr., CH-9445 Rebstein (CH)
(74) Vertreter: Reichert, Werner F., Dr.

(57) **Zusammenfassung**

Die Erfindung betrifft ein Ophthalmo-Operationsmikroskop (2), das ein integriertes Refraktometer (20) umfasst, welches die optischen Daten eines Patientenauges (1) 'in situ' während der Operation ermittelt und dem Chirurgen (29) bei Bedarf mitteilt.

## Beschreibung

Die Erfindung betrifft ein Ophthalmo-Operationsmikroskop mit einer Vorrichtung zur'in situ'-Ermittlung der optischen Daten eines Patientenauges während der Operation.

Bei Trübung oder starker Verformung der Linse eines Patientenauges werden heute üblicherweise die optischen Daten des Auges vor der Opera-tion mittels eines Refraktometers, beispielsweise mit dem 'The Welch Allyn Sure Sight ™' der Fa. Welch Allyn, NY 13153 (US), vergleiche Prospekt SM2275Rev. B, vor der Operation bestimmt. Aufgrund der gewonnenen Ergebnisse wird die Dimension der zu implantierenden IOL (Intra Ocular Lens) bestimmt. Dann wird im Laufe der Operation die IOL ins Auge eingesetzt. Nach dem Heilungsprozess - d.h. einige Tage danach - wird erneut mittels eines Refraktometers gemessen, die verbliebene Restfehlsichtigkeit bestimmt und bei Bedarf mittels einer Brille korrigiert.

Der Erfinder erkannte, dass diese bekannten Systeme nachteilig sind in Bezug auf die folgenden Punkte:
a) Dadurch, dass die optischen Daten vor der Operation und erst einige Tage nach der Operation bestimmt werden können, besteht für den Chirurgen keine Möglichkeit, Korrekturen während der Operation vorzunehmen, d.h. es bleibt unter Umständen eine beträchtliche noch zu korrigierende Fehlsichtigkeit des Patientenauges übrig.
b) Dem Chirurgen ist es ohne Unterbrechung der Operation nicht möglich, während der Operation die optischen Daten des Patientenauges zu bestimmen und damit entsprechende Korrekturmaßnahmen einzuleiten, um die Restfehlsichtigkeit zu minimieren.
c) Es besteht somit keine direkte Qualitätskontrolle während der Operation.

Aufgabe der Erfindung ist es nun, eine Vorrichtung zu schaffen, welche es ermöglicht, die optischen Daten eines Patientenauges während der Operation zu bestimmen, ohne den Operationsvorgang selbst unterbrechen zu müssen.

Gelöst wird diese Aufgabe durch die nachfolgend beschriebene Vorrichtung:

In ein standardmäßiges Operationsmikroskop wird ein Refraktometer (Auto-Refractive-Generator und -Analysator) integriert, welches die Bestimmung der optischen Daten des Patientenauges 'in situ' - d.h. während der Operation - erlaubt. Dies geschieht vorzugsweise auf folgendem Wege:

Das von einem Refraktometer generierte Licht wird über eine Optik und beispielsweise einen Strahlenteiler in den Hauptstrahlengang eines Mikroskops eingespiegelt und auf das Patientenauge projiziert. Das von der Netzhaut des Patientenauges reflektierte Refraktometer-Licht wird wiederum mittels beispielsweise eines Strahlenteilers aus dem Strahlengang des Mikroskops ausgespiegelt und über eine Optik auf eine Analyseeinheit des Refraktometers abgebildet. Das Refraktometer bzw. dessen Auswertungseinheit bestimmt damit laufend die Qualität der Korrektur durch die ins Auge implantierte Linse und teilt dies über ein Display, eine Vorrichtung zur Einspiegelung von Daten und/oder über Signalton/Signallicht dem Operateur mit. Die Ein- und Ausspiegelung der Refraktometer-Strahlengänge erfolgt beispielsweise direkt oberhalb des Zooms oder zwischen dem Zoom und dem Hauptobjektiv. Es lassen sich mit dem Grundprinzip der Erfindung verschiedene Varianten aufbauen.

Durch die vorgängig beschriebene Vorrichtung werden die folgenden Verbesserungen erreicht:
- Durch die 'in situ'-Ermittlung der optischen Daten des Patientenauges während der Operation lassen sich durch den Chirurgen frühzeitig Gegenmaßnahmen bei Abweichungen von den Soll-Werten einleiten.
- Durch die frühzeitige Messung und Korrektur von potentiellen Abweichungen lässt sich die übrigbleibende Fehlsichtigkeit nach dem Heilungsprozess minimieren.
- Insgesamt kann damit sowohl die Qualität des Operations-Ergebnisses wie auch die Gefahr einer größeren Abweichung während der Operation auf ein Minimum reduziert werden.
- Gemäß einer Weiterentwicklung der Erfindung kann der Chirurg bei Abweichungen über einem festzulegenden Schwellenwert während der Operation mittels eines Warnmechanismus - beispielsweise eines Signaltons und/oder einer Blinkleuchte und/oder eines Einspiegelungmoduls - auf eine akute Gefahr aufmerksam gemacht werden.

Anhand von schematischen Zeichnungen wird die Erfindung näher erläutert. Es zeigen dabei:
- Fig.1:: eine erfindungsgemäße Integration eines Refraktometers in ein Operationsmikroskop;
- Fig.2:: das prinzipielle Schaltbild aller Komponenten.

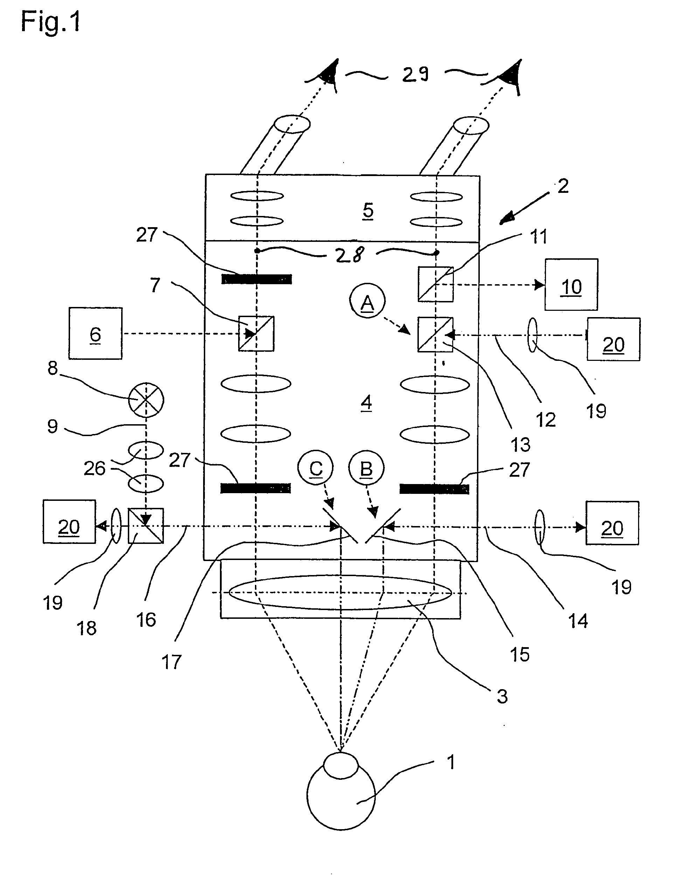

Fig.1 zeigt das vom Patientenauge 1 ausgehende Licht, welches über ein Hauptobjektiv 3 und über einen Zoom 4 auf den Tubus 5 mit Okularen geleitet wird. Im rechten Hauptstrahlengang des Mikroskops 2 ist beispielsweise zwischen dem Zoom 4 und dem Tubus 5 ein Strahlenteiler 11 angeordnet, welcher das Licht für eine Video-Dokumentationsvorrichtung 10 ausspiegelt. Im linken Hauptstrahlengang ist beispielsweise zwischen dem Zoom 4 und dem Tubus 5 ein Strahlenteiler 7 zur Einspiegelung der von einer Bildeinspiegelungs-Vorrichtung 6 generierten Daten in den Betrachtungsbereich des Chirurgen angeordnet. Zusätzlich ist ein von einer Beleuchtung 8 ausgehender Beleuchtungsstrahlengang 9 ersichtlich, welcher das Licht über einen Strahlenteiler 18 sowie einen Umlenkspiegel 17 auf das Patientenauge 1 projiziert.

Das von einem Refraktometer 20 generierte Licht wird erfindungsgemäß über eine Optik 19 für den Refraktometer-Strahlengang 12 an der Position A - direkt über dem Zoom 4 gelegen - mittels des Strahlenteilers 13 beispielsweise in den rechten Hauptstrahlengang des Mikroskops 2 eingeblendet. Dieses Licht wird über den Zoom 4 und das Hauptobjektiv 3 auf der Hornhaut des Patientenauges 1 abgebildet. Das von dort reflektierte Refraktometer-Licht wird wiederum über das Hauptobjektiv 3 und den Zoom 4 auf den Strahlenteiler 13 geleitet, welcher das Refraktometer-Licht ausspiegelt und über die Optik 19 für den Refraktometer-Strahlengang 12 zur Analyse auf die Messeinheit des Refraktometers 20 leitet.

Als Variante wird das Refraktometer-Licht an der Position B, die direkt über dem Hauptobjektiv 3 liegt, über einen Umlenkspiegel 15 für den Refraktometer-Strahlengang 14 auf das Patientenauge 1 projiziert und registriert.

Als weitere Variante wird das Refraktometer-Licht an der Position C, die zentral über dem Hauptopjektiv 3 liegt, mittels des Umlenkspiegels 17 für den Refraktometer-Strahlengang 16 und die Beleuchtung 9 mit der Beleuchtungsoptik 26 auf den Strahlenteiler 18 umgelenkt, welcher den Beleuchtungsstrahlengang 9 und den Refraktometer-Strahlengang 16 zusammenführt. Der Refraktometer-Strahlengang wird über eine Optik 19 für den Refraktometer-Strahlengang auf das Refraktometer 20 geleitet.

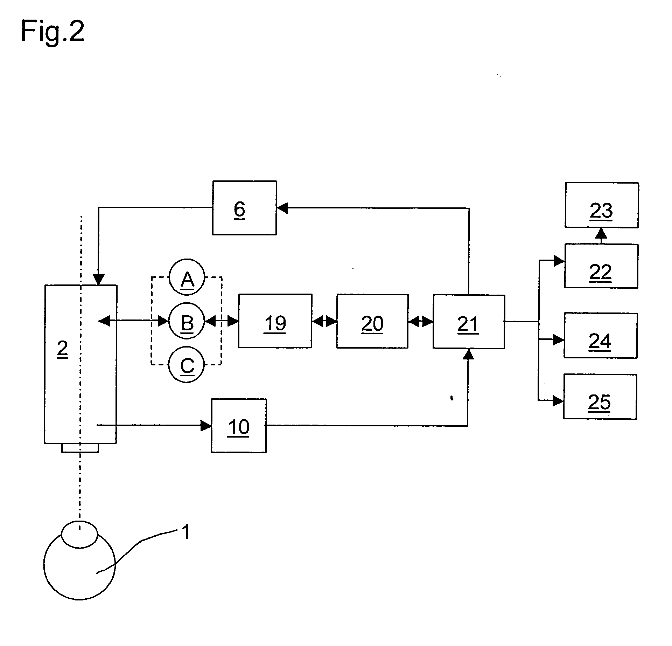

Fig.2 zeigt das prinzipielle Schaltbild aller Komponenten: Die vom Refraktometer 20 gemessenen Daten werden über eine Auswertungsund Steuereinheit 21 verarbeitet und gespeichert. Die Datenausgabe erfolgt beispielsweise über einen Monitor 22. Dort wird das Patientenauge 1 abgebildet, wahlweise mit den vom Refraktometer gemessenen optischen Daten des Patientenauges und eventueller weiterer Patientendaten. Diese können wiederum über einen Bildprinter 23 oder einen reinen Datenprinter 24 ausgedruckt oder in die Patientendatei 25 übermittelt werden. Die für die Operation notwendigen Patientendaten werden bei Bedarf aus der Patientendatei 25 in die Auswertungs- und Steuereinheit 21 übertragen.

Die Auswertungs- und Steuereinheit 21 bereitet die Daten der Video-Dokumentations-Vorrichtung 10, vorzugsweise einer Digital-Kamera, auf und stellt sie über eine Bildeinspiegelungs-Vorrichtung 6 zur Einspiegelung von Daten dem Operateur (29) im Mikroskopbild zur Verfügung.

Mit Hilfe von Shuttern 27 - vgl. Fig. 1 - kann beispielsweise derjenige Beobachterstrahlengang (28), in den die Daten nicht eingespiegelt werden, abgedunkelt werden. Dies ist wahlweise aber auch in demjenigen Strahlengang möglich, in den die Daten eingespiegelt werden. Dies ermöglicht eine bessere und kontrastreichere Datendarstellung. Hierfür bieten sich die Möglichkeiten an, die in den Patentanmeldungen der Anmelderin DE 10118703 und DE 10118702 offenbart ist.

### Bezugszeichenliste

- 1: Patientenauge
- 2: Operationsmikroskop
- 3: Hauptobjektiv
- 4: Zoom
- 5: Tubus mit Okularen
- 6: Bildeinspiegelungs-Vorrichtung
- 7: Strahlenteiler für (6)
- 8: Beleuchtung
- 9: Beleuchtungsstrahlengang
- 10: Video-Dokumentations-Vorrichtung
- 11: Strahlenteiler für (10)
- 12: Refraktometer-Strahlengang an der Position A (Ein-/Ausspiegelung)
- 13: Strahlenteiler für (12)
- 14: Refraktometer-Strahlengang an der Position B (Ein-/Ausspiegelung)
- 15: Umlenkspiegel für (14)
- 16: Refraktometer-Strahlengang an der Position C (Ein-/Ausspiegelung)
- 17: Umlenkspiegel für (16) und (8)
- 18: Strahlenteiler für (16)
- 19: Optik für Refraktometer-Strahlengang (Positionen A, B und/oder C)
- 20: Refraktometer
- 21: Auswertungs- und Steuereinheit
- 22: Monitor
- 23: Bildprinter
- 24: Datenprinter
- 25: Patientendatei
- 26: Beleuchtungsoptik
- 27: Shutter (Blende(n))
- 28: (Teil-)Strahlengänge für (29)
- 29: Beobachter (Chirurg)

## Patentansprüche

1. Ophthalmo-Operationsmikroskop (2) zur Beobachtung eines Patientenauges (1) während der Operation, **dadurch gekennzeichnet, dass** das Mikroskop (2) ein integriertes Refraktometer (20) (Auto-Refractive-Generator und -Analysator) umfasst, welches die optischen Daten des Patientenauges 'in situ' während der Operation ermittelt und dem Chirurgen bei Bedarf mitteilt.

2. Operationsmikroskop nach Anspruch 1, **dadurch gekennzeichnet, dass** die Einspiegelung der vom Refraktometer (20) generierten Strahlung an mindestens einer der drei Positionen
(A): zwischen Zoom (4) und Tubus (5) und/oder
(B): zwischen Hauptoptikiv (3) und Zoom (4) und/oder
(C): unmittelbar oberhalb des Hauptoptikivs (3) gelegen,
erfolgt.

3. Operationsmikroskop nach einem der vorhergehenden Ansprüche, **dadurch gekennzeichnet, dass** die Ausspiegelung der am Patientenauge (1) reflektierten Refraktometer-Strahlung an mindestens einer der drei Positionen
(A): zwischen Zoom (4) und Tubus (5) und/oder
(B): zwischen Hauptoptik (3) und Zoom (4) und/oder
(C): unmittelbar oberhalb des Hauptoptikivs (3) gelegen,
erfolgt.

4. Operationsmikroskop nach einem der vorhergehenden Ansprüche, **dadurch gekennzeichnet, dass** für die Ein- und Ausspiegelung der Refraktometer-Strahlengänge (12, 14, 16) Strahlenteiler (13, 18) bzw. Prismen oder Spiegel (15, 17) vorgesehen sind.

5. Operationsmikroskop nach einem der vorhergehenden Ansprüche, **dadurch gekennzeichnet, dass** die für die Ausspiegelung der Refraktometer-Strahlengänge bestimmten Strahlenteiler (13, 18) ausschließlich bestimmte Lichtwellenlängenbereiche transmittieren bzw. reflektieren, beispielsweise IR-Licht.

6. Operationsmikroskop nach einem der vorhergehenden Ansprüche, **dadurch gekennzeichnet, dass** die Adaptierung der Optik des Refraktometers (20) an die Optik des Operationsmikroskops (2) mittels einer dazwischen liegenden Optik (19) für die Refraktometer-Strahlengänge (12, 14, 16) erfolgt, welche beispielsweise aus Linsen, Prismen und/oder Spiegeln besteht.

7. Operationsmikroskop nach einem der vorhergehenden Ansprüche, **dadurch gekennzeichnet, dass** es mit mindestens einer Video-Dokumentations-Vorrichtung (10) ausrüstbar ist, welche das Patientenauge (1) aufnimmt und gegebenenfalls über einen Monitor (22) darstellbar macht.

8. Operationsmikroskop nach einem der vorhergehenden Ansprüche, **dadurch gekennzeichnet, dass** es mit mindestens einer Bildeinspiegelungs-Vorrichtung (6) ausrüstbar ist, die im Betriebszustand und bei Bedarf die für die Operation notwendigen Daten - wie Refraktometer-, Patienten- und/oder Video-Daten - ins Blickfeld des Chirurgen (29) einspiegelt.

9. Operationsmikroskop nach einem der vorhergehenden Ansprüche, **dadurch gekennzeichnet, dass** das Refraktometer (20) bidirektional mit dem Autofokus des Operationsmikroskops (2) koppelbar ist.

10. Operationsmikroskop nach einem der vorhergehenden Ansprüche, **dadurch gekennzeichnet, dass** die mittels des Refraktometers (20) bestimmten Patientendaten über eine Auswertungseinheit (21) in eine Patientendatei (25) übermittelbar und/oder auf einen Datenprinter (24) ausgebbar sind.

11. Operationsmikroskop nach einem der vorhergehenden Ansprüche, **dadurch gekennzeichnet, dass** die für die Operation relevanten Patientendaten im Betriebszustand automatisch und - vorzugsweise -elektronisch aus der Patientendatei (25) in die Auswertungs- und Steuereinheit (21) übernehmbar sind.

12. Operationsmikroskop nach einem der vorhergehenden Ansprüche, **dadurch gekennzeichnet, dass** zur besseren Sichtbarmachung der eingespiegelten Daten Shutter (27) wahlweise in die Strahlengänge (28) für den Beobachter (29) einbringbar sind.
